# EUROPEAN PATENT APPLICATION

(11) **EP 4 371 492 A1**
(43) Date of publication of application: **22.05.2024**
(21) Application number: 22953377.3
(22) Date of filing: 18.08.2022
(51) Int. Cl.: A61B 5/346, A61B 5/00, G16H 50/50, G16H 50/70, G16H 50/20, G06N 20/00

(54) **DEEP LEARNING-BASED HEALTH CONDITION PREDICTION SYSTEM USING PLURALITY OF ELECTROCARDIOGRAMS**

(71) Applicant: Medicalai Co., Ltd., Seoul 06302 (KR)
(72) Inventor: KWON, Joon Myoung, Seoul 06629 (KR)
(74) Representative: Isarpatent
(86) International application number: PCT/KR2022/012364
(87) International publication number: WO 2024/038930

(57) **Abstract**

The present invention discloses a system for predicting a health condition based on deep learning by using a plurality of electrocardiograms according to an embodiment of the present invention includes: an electrocardiogram measurement unit (110) configured to acquire N pieces of electrocardiogram data by measuring electrocardiograms of the same examinee at different times; a prediction unit (120) configured to generate disease information predicting the presence/absence, progress and degree of a disease by integrating semantic feature values or output values from the N pieces of electrocardiogram data provided from the electrocardiogram measurement unit (110) through a diagnostic algorithm previously constructed by being trained on datasets of N pieces of electrocardiogram data at different times and diseases corresponding to the electrocardiogram data; and an input unit (130) configured to convert the N pieces of electrocardiogram data input from the electrocardiogram measurement unit (110) and input them to the prediction unit (120).

## Description

### Technical Field

The present invention relates to a system for predicting a health condition based on deep learning by using a plurality of electrocardiograms that may improve the accuracy of measurement, diagnosis, examination, and prediction of a health condition by comparing and analyzing a plurality of electrocardiograms of the same examinee, taken at different times, based on deep learning and may analyze and predict the presence/absence, progress, and degree of disease in a time-series manner.

### Background Art

As is well known,
an electrocardiogram is a graphical record of electrical potentials related to heartbeat on the surface of a human body. Electrocardiograms include not only standard 12-lead electrocardiograms but also exercise stress electrocardiograms and Holter electrocardiograms.

Such electrocardiograms are used for the examination and diagnosis of circulatory diseases, and have the advantages of being simple, relatively inexpensive, non-invasive, and easily recorded repeatedly.

Meanwhile, as for standard 12-lead electrocardiograms used in hospitals, six electrodes are attached to the front of the chest, three electrodes are attached to the limbs, 12-lead electrocardiogram information is all collected and determined in an integrated manner, and then a disease is diagnosed.

In this case, 12-lead electrocardiograms record electrical potentials of the heart in 12 electrical directions centered on the heart. Through this, the disease of the heart confined to one area can be diagnosed.

However, changes in the electrocardiogram do not apply equally to all examinees. In other words, the shape of an electrocardiogram may appear like an examinee has a disease even when he or she does not have the disease at all. Although another examinee has a disease, the shape of his/her electrocardiogram may appear like he or she does not have the disease.

Therefore, there is a demand for technology that can improve the accuracy of measurement, diagnosis, examination, and prediction of a health condition by comparing and analyzing a plurality of electrocardiograms of the same examinee taken at different times.

### Disclosure

### Technical Problem

The technical problem to be overcome by the spirit of the present invention is to provide a system for predicting a health condition based on deep learning by using a plurality of electrocardiograms that may improve the accuracy of measurement, diagnosis, examination, and prediction of a health condition by comparing and analyzing a plurality of electrocardiograms of the same examinee, taken at different times, based on deep learning.

### Technical Solution

In order to overcome the above-described problem, an embodiment of the present invention provides a system for predicting a health condition based on deep learning by using a plurality of electrocardiograms, the system including: an electrocardiogram measurement unit configured to acquire N pieces of electrocardiogram data by measuring electrocardiograms of the same examinee at different times having a time difference therebetween; a prediction unit configured to generate disease information predicting the presence/absence, progress and degree of a disease by integrating semantic feature values or output values from the N pieces of electrocardiogram data provided from the electrocardiogram measurement unit through a diagnostic algorithm previously constructed by being trained on datasets of N pieces of electrocardiogram data at different times and diseases corresponding to the electrocardiogram data; and an input unit configured to convert the N pieces of electrocardiogram data input from the electrocardiogram measurement unit and input them to the prediction unit.

In this case, the input unit may integrate the N pieces of electrocardiogram data, input from the electrocardiogram measurement unit, into a single piece of electrocardiogram data.

Furthermore, the input unit may integrate the N pieces of electrocardiogram data on a per electrocardiogram data unit basis.

Furthermore, the input unit may classify the N pieces of electrocardiogram data for respective leads, and may integrate the electrocardiogram data, classified for the respective leads, for the respective leads.

Furthermore, the input unit may classify the N pieces of electrocardiogram data for respective leads, may divide them into bit units, and may pair and integrate bit units for each same lead.

Moreover, the prediction unit may generate the disease information by extracting semantic feature values from the N pieces of electrocardiogram data provided from the input unit, analyzing the semantic feature values in an integrated manner, and comparing and analyzing the N pieces of electrocardiogram data.

### Advantageous Effects

According to the present invention, there are effects in that the accuracy of measurement, diagnosis, examination, and prediction of a health condition may be improved by comparing and analyzing a plurality of electrocardiograms of the same examinee, taken at different times, based on deep learning and the presence/absence, progress, and degree of disease may be analyzed and predicted in a time-series manner.

### Description of Drawings

FIG. 1 shows the configuration of a system for predicting a health condition based on deep learning by using a plurality of electrocardiograms according to an embodiment of the present invention;
FIG. 2 illustrates an electrocardiogram integration method using the system for predicting a health condition based on deep learning by using a plurality of electrocardiograms shown in FIG. 1; and
FIG. 3 illustrates a flowchart of a prediction method using the system for predicting a health condition based on deep learning by using a plurality of electrocardiograms shown in FIG. 1.

### Mode for Invention

Embodiments of the present invention having the above-described features will be described in detail below with reference to the accompanying drawings.

A system for predicting a health condition based on deep learning by using a plurality of electrocardiograms according to an embodiment of the present invention includes: an electrocardiogram measurement unit 110 configured to acquire N pieces of electrocardiogram data by measuring electrocardiograms of the same examinee at different times having a time difference therebetween; a prediction unit 120 configured to generate disease information predicting the presence/absence, progress and degree of a disease by integrating semantic feature values or output values from the N pieces of electrocardiogram data provided from the electrocardiogram measurement unit 110 through a diagnostic algorithm previously constructed by being trained on datasets of N pieces of electrocardiogram data at different times and diseases corresponding to the electrocardiogram data; and an input unit 130 configured to convert the N pieces of electrocardiogram data input from the electrocardiogram measurement unit 110 and input them to the prediction unit 120. The gist of the system for predicting a health condition based on deep learning by using a plurality of electrocardiograms is to more accurately measure, diagnose, examine, and predict a health condition from a plurality of pieces of electrocardiogram data of the same examinee at different times having a time difference therebetween.

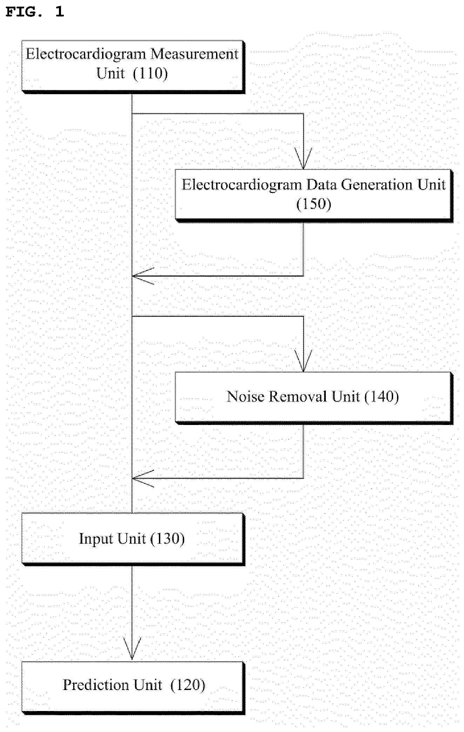

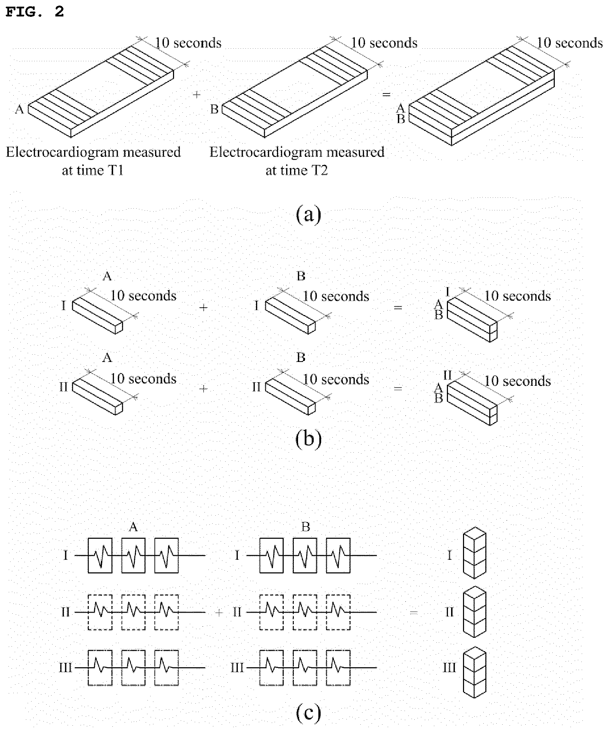

The system for predicting a health condition based on deep learning by using a plurality of electrocardiograms, which is configured as described above, will be described in detail below with reference to FIGS. 1 and 2 as follows.

First, the electrocardiogram measurement unit 110 acquires N pieces of electrocardiogram data by measuring electrocardiograms of the same examinee at different times having a time difference therebetween.

In this case, the electrocardiogram measurement unit 110 may measure N pieces of electrocardiogram data at two or more respective measurement times by measuring electrocardiogram data of the same examinee at different times having a large time difference, such as a monthly, quarterly, or yearly time difference, therebetween.

Meanwhile, the electrocardiogram measurement unit 110 may include a wearable electrocardiogram patch, a smartwatch or a 6-lead electrocardiogram bar for short-term measurement capable of contact or contactless electrocardiogram measurement during daily life, or an electrocardiogram device installed in a medical institution, may measure asynchronous or synchronous electrocardiograms, and may provide them to the input unit 130.

Next, through a diagnostic algorithm previously constructed by being trained on datasets of N pieces of electrocardiogram data of each of the same examinees at different times and diseases corresponding to the electrocardiogram data, which are big data accumulated in servers of medical institutions, the prediction unit 120 generates disease information predicting the presence/absence, progress, and degree of a disease by integrating semantic feature values or output values from the N pieces of electrocardiogram data provided from the electrocardiogram measurement unit 110.

In this case, the semantic feature values are spatial time-series feature values that are extracted from the electrocardiogram data. Based on these, the prediction unit 120 may measure, diagnose, examine, and predict a health condition at the current time at which the electrocardiogram data was last measured or a future health condition by comparing and analyzing the N pieces of electrocardiogram data taken at different times.

Meanwhile, the prediction unit 120 may generate time-series disease information by comparing and analyzing the N pieces of electrocardiogram data taken at different times in such a manner as to extract semantic feature values from the N pieces of electrocardiogram data taken at different times provided from the input unit 130, which will be described later, and then analyze the semantic feature values in an integrated manner.

Furthermore, as a method of analyzing extracted semantic feature values in an integrated manner, one of various deep learning algorithms such as CNN, LSTM, RNN, and MLP may be used, and one of machine learning methods such as logistic regression, a principle-based model, a random forest, and a support vector machine may also be used.

Next, the input unit 130 converts the N pieces of electrocardiogram data input from the electrocardiogram measurement unit 110 and inputs them to the prediction unit 120.

In this case, the input unit 130 may integrate the N pieces of electrocardiogram data of the same examinee at different times, input from the electrocardiogram measurement unit 110, into a single piece of electrocardiogram data, and may input it to the prediction unit 120.

For example, as illustrated in FIG. 2(a), the input unit 130 may integrate the N pieces of electrocardiogram data at different times into the same time-axis length on a per electrocardiogram data unit in their entirety. Meanwhile, when the N pieces of electrocardiogram data at different times are not measured for the same time-axis length, they may be cut in accordance with the minimum time-axis length, or an electrocardiogram for a required length for the remaining electrocardiogram data is generated in accordance with the maximum time-axis length and then overall lengths are made equal through a deep learning algorithm.

Alternatively, as illustrated in FIG. 2(b), the input unit 130 may determine the individual characteristics of electrocardiogram data for individual leads for N pieces of electrocardiogram data at different times T1 and T2, may classify the electrocardiogram data according to the determined characteristics, may integrate the classified electrocardiogram data for the individual leads on a per lead basis, and may input the integrated electrocardiogram data to the prediction unit 120.

Alternatively, as illustrated in FIG. 2(c), the input unit 130 may classify the N pieces of electrocardiogram data at different times on a per lead basis, may divide the classified electrocardiogram data for the individual leads into bit units, may pair and integrate bit units of the same lead, and may input the integrated bit units to the prediction unit 120.

Meanwhile, there may be further included a noise removal unit 140 in order to further increase the reliability of the disease information generated by the prediction unit 120 by minimizing noise in the electrocardiogram data provided from the electrocardiogram measurement unit 110 to the input unit 130. For example, through a deep learning algorithm constructed by being pre-trained on datasets of electrocardiogram data for individual leads having less noise and the unique styles of the electrocardiogram data for individual leads based on a large amount of electrocardiogram data, such as the standard 12-lead electrocardiogram data accumulated in medical institutions, the noise removal unit 140 may extract a corresponding unique style from the electrocardiogram data measured by the electrocardiogram measurement unit 110 with the characteristics of an examinee and the characteristics of a measurement method reflected therein, and may perform conversion into and generate electrocardiogram data of a specific lead style containing no noise through the extracted unique style.

Furthermore, the noise removal unit 140 may identify the unique style of each electrocardiogram data for each lead with high accuracy with the characteristics of the examinee attributable to age, gender, disease, etc. and the characteristics of the measurement method attributable to the attachment locations of electrodes, an electrocardiogram device, etc. reflected therein, and may perform conversion into and generate electrocardiogram data for each lead based on the identified unique style more accurately.

Furthermore, when electrocardiogram data of some lead or section contains a lot of noise in electrocardiogram data or measurement is not performed appropriately due to the loss of electrode contact upon measurement performed by the electrocardiogram measurement unit 110, a noise-free electrocardiogram may be generated and missing electrocardiogram data may be filled in through the electrocardiogram data generation unit 150, so that a health condition can be measured, diagnosed, examined, and predicted more accurately.

Furthermore, the prediction unit 120 may diagnose and predict diseases of the circulatory system, endocrine, nutritional and metabolic diseases, neoplastic diseases, mental and behavioral disorders, diseases of the nervous system, diseases of the eyes and their appendages, diseases of the ears and mastoids, diseases of the respiratory system, diseases of the digestive system, diseases of the skin and skin tissue, diseases of the musculoskeletal system and compressive tissue, diseases of the genitourinary system, pregnancy, childbirth and postpartum diseases, and congenital anomalies, deformities and chromosomal abnormalities.

In addition, through the prediction unit 120, the damage caused by physical trauma may be identified, the prognosis thereof may be checked and the pain thereof may be measured, the risk of death or worsening attributable to trauma may be predicted, concurrent complications may be detected or predicted, and specific conditions that appear before or after birth may also be identified.

Furthermore, through the prediction unit 120, for the healthcare field, the health condition of an examinee, which may lead to services such as aging, sleep, weight, blood pressure, blood sugar, oxygen saturation, metabolism, stress, tension, fear, drinking, smoking, problem behavior, lung capacity, exercise volume, pain management, obesity, body mass, body composition, diet, type of exercise, lifestyle pattern recommendations, emergency management, chronic disease management, medication prescription, test recommendation, checkup recommendation, nursing, telehealth management, telemedicine, vaccination, and post-vaccination management, may be measured, diagnosed, examined, and predicted.

Not only health conditions attributable to the individual diseases described above but also complex health conditions may be measured, diagnosed, examined and predicted, the worsening or alleviation of the health condition of an examinee may be predicted, short-term and long-term prognoses may be predicted, and the state of metastasis or complication from one disease to another may be predicted. The improvement or deterioration of health conditions attributable to specific medicines and the analysis and prediction of electrocardiograms may be trained. A specific medicine may be recommended according to a health condition.

Accordingly, according to the above-described configuration of the system for predicting a health condition based on deep learning by using a plurality of electrocardiograms, a disease may be diagnosed more accurately by comparing the electrocardiogram taken at a previous time and the electrocardiogram taken at a later time with each other. For example, when the electrocardiogram at the previous time showed findings of myocardial infarction due to ST-segment elevation or the like but the examination was normal and the electrocardiogram at the later time, e.g., one year later, showed findings of myocardial infarction due to the same ST-segment elevation as before, the possibility of myocardial infarction is low. In other words, since it is impossible to have a myocardial infarction for a long period of one year, an examinee should be regarded as a normal patient.

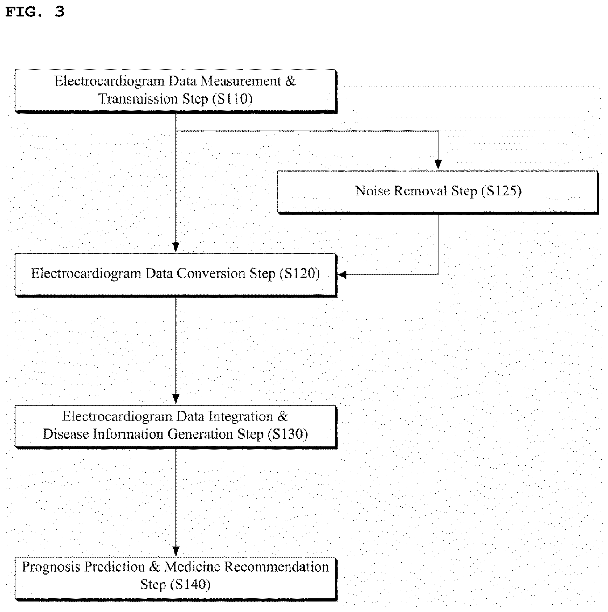

Meanwhile, FIG. 3 illustrates a flowchart of a prediction method using the system for predicting a health condition based on deep learning by using a plurality of electrocardiograms shown in FIG. 1. The prediction method will be described in detail with reference to this drawing.

First, through the electrocardiogram measurement unit 110, N pieces of electrocardiogram data may be acquired by measuring electrocardiograms of the same examinee at different times having a time difference therebetween and provided to the input unit 130 in step S110.

In this case, the electrocardiogram measurement unit 110 may measure N pieces of electrocardiogram data at two or more respective measurement times by measuring the electrocardiogram data of the same examinee at different times having a large time difference, such as a monthly, quarterly, or yearly time difference, therebetween.

Meanwhile, the electrocardiogram measurement unit 110 may include a wearable electrocardiogram patch, a smartwatch or a 6-lead electrocardiogram bar for short-term measurement capable of contact or contactless electrocardiogram measurement during daily life, or an electrocardiogram device installed in a medical institution, may measure asynchronous or synchronous electrocardiograms, and may provide them to the input unit 130.

Thereafter, N pieces of electrocardiogram data from the electrocardiogram measurement unit 110 are converted and input to the prediction unit 120 through the input unit 130 in step S120.

In this case, the input unit 130 may integrate the N pieces of electrocardiogram data of the same examinee at different times, input from the electrocardiogram measurement unit 110, into a single piece of electrocardiogram data and input it to the prediction unit 120.

For example, as illustrated in FIG. 2(a), the input unit 130 may integrate the N pieces of electrocardiogram data at different times into the same time-axis length on a per electrocardiogram data unit in their entirety. Meanwhile, when the N pieces of electrocardiogram data at different times are not measured for the same time-axis length, they may be cut in accordance with the minimum time-axis length, or an electrocardiogram for a required length for the remaining electrocardiogram data is generated in accordance with the maximum time-axis length and then overall lengths are made equal through a deep learning algorithm.

Alternatively, as illustrated in FIG. 2(b), the input unit 130 may determine the individual characteristics of electrocardiogram data for individual leads for N pieces of electrocardiogram data at different times T1 and T2, may classify the electrocardiogram data according to the determined characteristics, may integrate the classified electrocardiogram data for the individual leads on a per lead basis, and may input the integrated electrocardiogram data to the prediction unit 120.

Alternatively, as illustrated in FIG. 2(c), the input unit 130 may classify the N pieces of electrocardiogram data at different times on a per lead basis, may divide the classified electrocardiogram data for the individual leads into bit units, may pair and integrate bit units of the same lead, and may input the integrated bit units to the prediction unit 120.

Meanwhile, there may be further included step S125 of further increasing the reliability of the disease information generated by the prediction unit 120 by minimizing noise in the electrocardiogram data, provided from the electrocardiogram measurement unit 110 to the input unit 130, through the noise removal unit 140. For example, through a deep learning algorithm constructed by being pre-trained on datasets of electrocardiogram data for individual leads having less noise and the unique styles of the electrocardiogram data for individual leads based on a large amount of electrocardiogram data, such as the standard 12-lead electrocardiogram data accumulated in medical institutions, the noise removal unit 140 may extract a corresponding unique style from the electrocardiogram data measured by the electrocardiogram measurement unit 110 with the characteristics of an examinee and the characteristics of a measurement method reflected therein, and may perform conversion into and generate electrocardiogram data of a specific lead style containing no noise through the extracted unique style.

Furthermore, the noise removal unit 140 may identify the unique style of each electrocardiogram data for each lead with high accuracy with the characteristics of the examinee attributable to age, gender, disease, etc. and the characteristics of the measurement method attributable to the attachment locations of electrodes, an electrocardiogram device, etc. reflected therein, and may perform conversion into and generate electrocardiogram data for each lead based on the identified unique style more accurately.

Furthermore, when electrocardiogram data of some lead or section contains a lot of noise in electrocardiogram data or measurement is not performed appropriately due to the loss of electrode contact upon measurement performed by the electrocardiogram measurement unit 110, a noise-free electrocardiogram may be generated and missing electrocardiogram data may be filled in through the electrocardiogram data generation unit 150, so that a health condition can be measured, diagnosed, examined, and predicted more accurately.

Next, through a diagnostic algorithm previously constructed by being trained on datasets of N pieces of electrocardiogram data of each of the same examinees at different times and diseases corresponding to the electrocardiogram data, which are big data accumulated in servers of medical institutions, disease information predicting the presence/absence, progress, and degree of a disease is generated by integrating semantic feature values or output values from the N pieces of electrocardiogram data provided from the electrocardiogram measurement unit 110 through the prediction unit 120 in step S130.

In this case, the semantic feature values are spatial time-series feature values that are extracted from the electrocardiogram data. Based on these, the prediction unit 120 may measure, diagnose, examine, and predict a health condition at the current time at which the electrocardiogram data was last measured or a future health condition by comparing and analyzing the N pieces of electrocardiogram data taken at different times.

Meanwhile, the prediction unit 120 may generate time-series disease information by comparing and analyzing N pieces of electrocardiogram data at different times in such a manner as to extract semantic feature values from N pieces of electrocardiogram data at different times provided from the input unit 130 to be described later and then analyze the semantic feature values in an integrated manner.

Furthermore, as a method of analyzing extracted semantic feature values in an integrated manner, one of various deep learning algorithms such as CNN, LSTM, RNN, and MLP may be used, and one of machine learning methods such as logistic regression, a principle-based model, a random forest, and a support vector machine may also be used.

Next, in step S140, through the prognosis prediction unit, not only health conditions attributable to the individual diseases described above but also complex health conditions may be measured, diagnosed, examined and predicted, the worsening or alleviation of the health condition of an examinee may be predicted, short-term and long-term prognoses may be predicted, and the state of metastasis or complication from one disease to another may be predicted. The improvement or deterioration of health conditions attributable to specific medicines and the analysis and prediction of electrocardiograms may be trained. A specific medicine may be recommended according to a health condition.

Therefore, in the present embodiment, the accuracy of measurement, diagnosis, examination, and prediction of a health condition may be improved by comparing and analyzing a plurality of electrocardiograms of the same examinee, taken at different times, based on deep learning. The presence/absence, progress, and degree of disease may be analyzed and predicted in a time-series manner.

The embodiments described in the present specification and the configurations shown in the drawings are only the most preferred embodiments of the present invention and do not represent the overall technical spirit of the present invention. Accordingly, it should be understood that at the time when the present application is filed, there may be various equivalents and modifications that can replace the above embodiments and/or configurations.

## Claims

1. A system for predicting a health condition based on deep learning by using a plurality of electrocardiograms, the system comprising:
an electrocardiogram measurement unit configured to acquire N pieces of electrocardiogram data by measuring electrocardiograms of a same examinee at different times having a time difference therebetween;
a prediction unit configured to generate disease information predicting a presence/absence, progress and degree of a disease by integrating semantic feature values or output values from the N pieces of electrocardiogram data provided from the electrocardiogram measurement unit through a diagnostic algorithm previously constructed by being trained on datasets of N pieces of electrocardiogram data at different times and diseases corresponding to the electrocardiogram data; and
an input unit configured to convert the N pieces of electrocardiogram data input from the electrocardiogram measurement unit and input them to the prediction unit.

2. The system of claim 1, wherein the input unit integrates the N pieces of electrocardiogram data, input from the electrocardiogram measurement unit, into a single piece of electrocardiogram data.

3. The system of claim 2, wherein the input unit integrates the N pieces of electrocardiogram data on a per electrocardiogram data unit basis.

4. The system of claim 2, wherein the input unit classifies the N pieces of electrocardiogram data for respective leads, and integrates the electrocardiogram data, classified for the respective leads, for the respective leads.

5. The system of claim 2, wherein the input unit classifies the N pieces of electrocardiogram data for respective leads, divides them into bit units, and pairs and integrates bit units for each same lead.

6. The system of claim 1, wherein the prediction unit generates the disease information by extracting semantic feature values from the N pieces of electrocardiogram data provided from the input unit, analyzing the semantic feature values in an integrated manner, and comparing and analyzing the N pieces of electrocardiogram data.
